(19) Europäisches Patentamt / European Patent Office / Office européen des brevets

(11) **EP 3 980 244 B1**

(12) **EUROPEAN PATENT SPECIFICATION**

(45) Date of publication and mention
of the grant of the patent:
**27.05.2026 Bulletin 2026/22**

(21) Application number: **20734854.1**

(22) Date of filing: **17.06.2020**

(51) International Patent Classification (IPC):
**B29C 64/118** (2017.01)    **B33Y 10/00** (2015.01)
**B33Y 70/00** (2020.01)    **C12Q 1/68** (2018.01)
**G06N 3/12** (2023.01)

(52) Cooperative Patent Classification (CPC):
(C-Sets available)
**B29C 64/118; B33Y 10/00; B33Y 70/00;**
**C12Q 1/68;** G06N 3/123    (Cont.)

(86) International application number:
**PCT/EP2020/066800**

(87) International publication number:
**WO 2020/254428 (24.12.2020 Gazette 2020/52)**

(54) **FUNCTIONAL MATERIALS WITH EMBEDDED MEMORY USING SEQUENCE-CONTROLLED POLYMER-BASED STORAGE**

FUNKTIONALE MATERIALIEN MIT EINGEBETTETEM SPEICHER UNTER VERWENDUNG VON SEQUENZGESTEUERTER POLYMERBASIERTER SPEICHERUNG

MATÉRIAUX FONCTIONNELS À MÉMOIRE INTÉGRÉE UTILISANT UN STOCKAGE À BASE DE POLYMÈRE À SÉQUENCE CONTRÔLÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.06.2019 US 201962863516 P**

(43) Date of publication of application:
**13.04.2022 Bulletin 2022/15**

(73) Proprietor: **ETH Zurich**
**8092 Zürich (CH)**

(72) Inventors:
• **GRASS, Robert, N.**
**8093 Zürich (CH)**
• **ERLICH, Yaniv**
**Las Vegas, NJ 89107 (US)**

(74) Representative: **E. Blum & Co. AG**
**Franklinturm**
**Hofwiesenstrasse 349**
**8050 Zürich (CH)**

(56) References cited:
**US-B2- 9 400 910**

• **ROBERT N. GRASS ET AL: "Robust Chemical Preservation of Digital Information on DNA in Silica with Error-Correcting Codes", ANGEWANDTE CHEMIE INTERNATIONAL EDITION, vol. 54, no. 8, 16 February 2015 (2015-02-16), pages 2552 - 2555, XP055194296, ISSN: 1433-7851, DOI: 10.1002/anie.201411378**
• **YANIV ERLICH ET AL: "DNA Fountain enables a robust and efficient storage architecture", SCIENCE, 3 March 2017 (2017-03-03), Washington, pages 950, XP055471220, Retrieved from the Internet <URL:10.1126/science.aaj2038> DOI: 10.1126/science.aaj2038**
• **KOCH JULIAN ET AL: "A DNA-of-things storage architecture to create materials with embedded memory", NATURE BIOTECHNOLOGY, GALE GROUP INC., NEW YORK, US, vol. 38, no. 1, 9 December 2019 (2019-12-09), pages 39 - 43, XP036983380, ISSN: 1087-0156, [retrieved on 20191209], DOI: 10.1038/S41587-019-0356-Z**

EP 3 980 244 B1

Remarks:
The complete document including Reference
Table(s) and the Sequence Listing(s) can be
downloaded from the EPO website

(52) Cooperative Patent Classification (CPC): (Cont.)

C-Sets
**C12Q 1/68, C12Q 2563/179, C12Q 2563/185**

**Description**

BRIEF SUMMARY

Embodiments of the present disclosure relate to sequence-controlled polymer storage, and more specifically, to sequence-controlled polymer storage in encapsulated materials. Further embodiments relate to methods for storing data in a 3D object using a data containing feedstock, to methods for recovering information from a 3 D object and to data storage media.

According to embodiments of the present disclosure, methods and systems for sequence-controlled polymer encoding (1st aspect), decoding (2nd aspect), and storage (3rd aspect) are provided. Accordingly, methods and systems for sequence-controlled polymer encoding, decoding, and storage are provided. In various embodiments, input data is encoded into one or more sequence controlled polymer, wherein encoding the input data comprises applying an error-correction code. The one or more sequence-controlled polymer are synthesized. The synthesized one or more sequence-controlled polymer are encapsulated in a plurality of particles. The plurality of particles are embedded into a feedstock. The feedstock is converted into a 3D object, preferably by 3D printing. In embodiments, these aspects are linked together by the special technical feature of a sequence controlled polymer comprising data and an error-correction code.

In a first aspect of the present disclosure, input data is encoded into one or more sequence controlled polymer, wherein encoding the input data comprises applying an error- correction code. The one or more sequence-controlled polymer are synthesized. The synthesized one or more sequence-controlled polymer are encapsulated in a plurality of particles. The plurality of particles are embedded into a feedstock.

In some embodiments, the feedstock comprises a filament adapted for 3D printing. In some embodiments, an object is 3D printed using the filament. In some embodiments, the data comprises a 3D model of the object. In some embodiments, the one or more sequence-controlled polymer comprises DNA. In some embodiments, encoding the input data further comprises applying a fountain code. In some embodiments, encoding the input data further comprises applying DNA Fountain encoding. In some embodiments, encoding the input data further comprises applying a Reed-Solomon code. In some embodiments, the plurality of particles comprises silica beads. In some embodiments, each of the plurality of particles has a diameter of at most IOOOnm. In embodiments, the plurality of particles are homogeneously distributed within the feedstock. In some embodiments, the plurality of particles have a concentration of at most IOOmg/kg within the feedstock. In some embodiments, the plurality of particles have a concentration of at most 2mg/g of DNA. In some embodiments, encapsulating the synthesized one or more sequence- controlled polymer comprises sol-gel synthesis. In some embodiments, the feedstock comprises thermo-polymer, i.e. a polymer having thermosoftening properties. In some embodiments, the feedstock comprises polycaprolactone (PCL), polymethylmethacrylate (PMMA), polylactic acid (PLA), acrylonitrile butadien styrene (ABS) or poly(lactic-co-glycolic acid) (PLGA) or combination thereof; specifically PCL.

In a second aspect, a plurality of particles are released from a thermo-polymer. One or more sequence-controlled polymer are extracted from the plurality of particles. The one or more sequence-controlled polymer are sequenced. Output data from the one or more sequence-controlled polymer are decoded, wherein the output data comprises a payload and an error correction code. The error correction is applied to the payload.

In some embodiments, releasing the plurality of particles comprises applying tetrahydrofuran to the thermo-polymer. In some embodiments, extracting the one or more sequence-controlled polymer comprises applying a buffered oxide etch to the plurality of particles. In some embodiments, the one or more sequence-controlled polymer comprises DNA. In some embodiments, the error correction code comprises a fountain code. In some embodiments, the error correction code comprises DNA Fountain encoding. In some embodiments, the error correction code comprises a Reed-Solomon code. In some embodiments, the plurality of particles comprises silica beads. In some embodiments, each of the plurality of particles has a diameter of at most IOOOnm. In embodiments, the plurality of particles are homogeneously distributed within the thermo-polymer. In some embodiments, the plurality of particles have a concentration of at most IOOmg/kg within the thermo-polymer. In some embodiments, the plurality of particles have a concentration of at most 2mg/g of DNA. In some embodiments, the thermo-polymer contains polycaprolactone (PCL), polymethylmethacrylate (PMMA), polylactic acid (PLA), acrylonitrile butadien styrene (ABS) or poly(lactic-co-glycolic acid) (PLGA) or combination thereof; specifically PCL.

In a third aspect, a data storage medium is provided. The data storage medium comprises a plurality of particles embedded in a thermo-polymer, with said plurality of particles being homogenously distributed within the thermo-polymer, and one or more sequence- controlled polymer encapsulated in the plurality of particles, wherein the one or more sequence- controlled polymer encodes predetermined data, the predetermined data comprising a payload and an error correction code.

In some embodiments, the data storage medium has a predetermined shape, wherein the predetermined data com prises a 3D model of the predetermined shape. In some embodiments, the one or more sequence-controlled polymer comprises DNA. In some embodiments, the error correction code comprises a fountain code. In some embodiments, the error correction code comprises DNA Fountain. In some embodiments, the error correction code comprises a Reed-Solomon code. In some embodiments, the plurality of particles comprises silica beads. In some embodiments, each of the plurality of particles has a diameter of at most lOOOnm. In embodiments, the plurality of particles are homogeneously distributed within the thermo-polymer. In some embodiments, the plurality of particles have a concentration of at most lOOmg/kg within the thermo-polymer. In some embodiments, the plurality of particles have a concentration of at most 2mg/g of DNA. In some embodiments, the thermo-polymer contains polycaprolactone (PCL), polymethylmethacrylate (PMMA), polylactic acid (PLA), acrylonitrile butadien styrene (ABS) or poly(lactic-co-glycolic acid) (PLGA) or combination thereof; specifically PCL.

Brief Description of the Drawings

**Fig. 1** is a plot of the robustness of encapsulated DNA (left block) and free DNA (right block) against various external factors (I both blocks from left to right: Reference, 60°C, ROS, Bleach) according to embodiments of the present disclosure. y-axis: Fraction of DNA recovered, logarithmic scale.

**Fig. 2** is a visualization of the DNA of things (DoT) process according to embodiments of the present disclosure.

**Fig. 3** is a visual comparison of the DoT process and parthenogenesis according to embodiments of the present disclosure. (1) Sequencing of DNA Library (2) Fountain code transformation of library into stl file; (3) 3D printing of identical object based on stl file, (4) integration of DNA into printing filament.

**Figs. 4A-C** are schematics of a sequence design for the DoT process according to experiments of embodiments of the present disclosure.

**Figs. 5A-B** are electron microscopy images of silica particle encapsulated DNA (SPED) according to embodiments of the present disclosure.

**Fig. 6** is a visualization of the hierarchical architecture of a 3D printed bunny containing information within its DNA according to embodiments of the present disclosure. From left to right:

- 105 bunny file units / g PCL
- 100 ppm particles in PCL
- 0.2 wt% DNA loading
- DNA library 12000 oligos * 145 nt (top); stl file size 100kB (bottom)

**Fig. 7** is a visualization of a step in a DoT process according to embodiments of the present disclosure.

**Fig. 8** is a visualization of the DoT process according to embodiments of the present disclosure.

(a) timing 10 min, hand-on time 10 min
(c) timing 4 days, hand-on time 3 h
(d) timing 2 h, hand-on time 2 h
(e) timing 2 h, hand-on time 20 min
(g) timing 1 h, hand-on time 1 h
(h; i) timing 20 h , hand-on time 3 h

**Fig. 9** is a visualization of a step in a DoT process according to embodiments of the present disclosure. Top: Parent (P), Bottom: Identical Progeny (IP).

**Fig. 10** is a plot of the file corruption rate for various generations of a DoT object according to embodiments of the present disclosure. x-axis: Generation ; y-axis: file corruption rate (%); dotted line: maximum restorable corruption rate

**Fig. 11** is a plot of the oligo coverage frequency for a generation of a DoT object according to embodiments of the present disclosure. x-axis: correct reads per oligo per million reads (OPM); y-axis: Frequency; Overdispersion 1.0; Mean 16.3

**Fig. 12** is a plot of the oligo coverage frequency for a generation of a DoT object according to embodiments of the present disclosure. x-axis: correct reads per oligo per million reads (OPM); y-axis: Frequency; Overdispersion 2.27; Mean 11.0

**Fig. 13** is a plot of the thermal stability of an encapsulated DNA file for different processes and temperatures according to embodiments of the present disclosure. x-axis: Process temperature (°C); y-axis: recovered DNA (ng/g of polymer)

**Fig. 14** is a plot of the qPCR cycle threshold for various SPED concentrations according to embodiments of the present disclosure. x-axis: log concentration (g/L); y-axis: cycle threshold; y = -3.563x + 3.685; $R^2$ = 0.99946.

**Fig. 15** depicts a computing node according to an embodiment of the present disclosure.

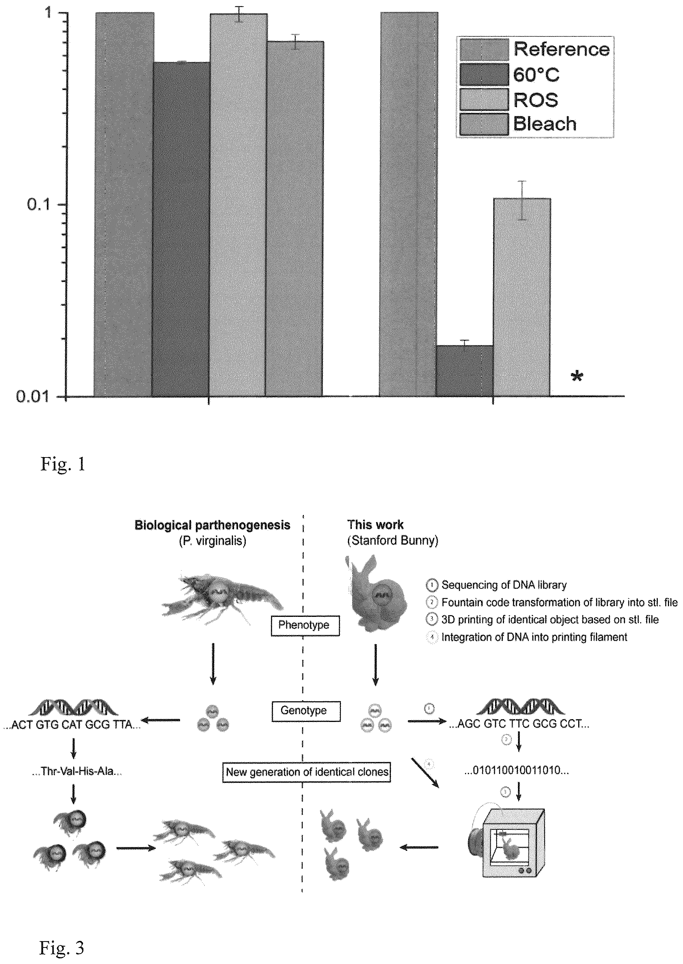

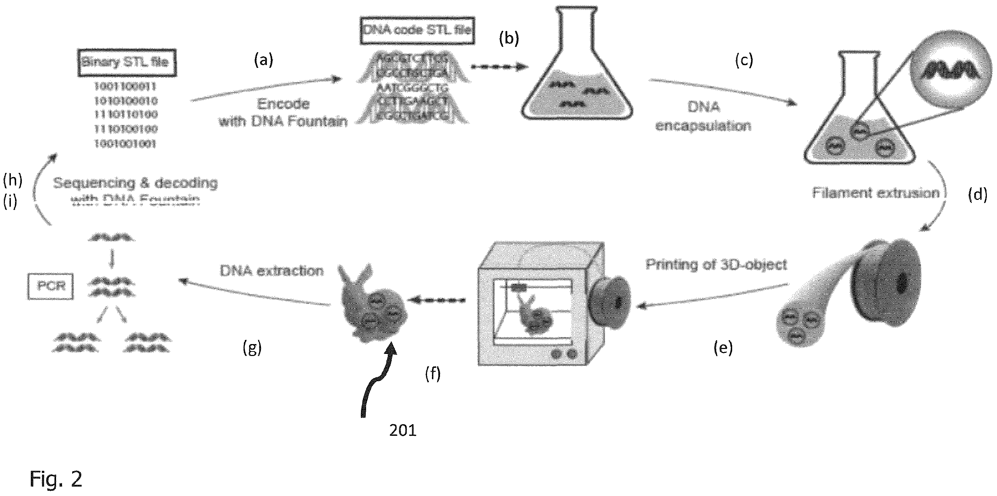

Figs. 2, 7 - 12 are also post - published in Koch et al, nature biotechnology, 38, Jan 2020, 39 -43, figures 1 and 2.

Description

US patent no. US9400910B2 describes a computer implemented method and apparatus for storing and retrieving data embedded in a 3D symbol matrix printed on the surface of a 3D object. The 3D symbol matrix printed concurrently with the object on the surface of said object is representative of data and enables storage of higher amount of data than the existing 2D barcodes, improvement of the durability of data compared with a surface label and improvement of data security compared with the data security achieved with 2D barcodes. DNA storage offers substantial information density and exceptional half-life. To allow for the chemical preservation of the information, Grass et al. (Angew. Chem. Int. Ed. 2015, 54, 2552-2555) proposes encapsulating the DNA in an inorganic matrix. Erlich et al. (Science 355, 950-954 (2017)) reports on a storage strategy, called DNA Fountain, which approaches the information capacity per nucleotide. The present disclosure provides for a storage architecture, dubbed the DNA of things (DoT), which creates materials with memory, which in some embodiment is immutable. The DoT framework records data directly onto DNA molecules, encapsulates them in nanometer silica beads, and fuses the beads into a feedstock, such as a filament containing (i.e. comprising or consisting of thermo-polymer, e.g. a Polycaprolactone (PCL) filament. The filament containing thermos-polymer, e.g the PCL filament, is used as a 3D printing feedstock to create objects with embedded data in any desired shape. Suitable objects include custom manufactured engineering parts, medical and dental implants, transparent or translucent windows, or packaging materials. In some embodiments, DoT is used to create a 3D-printed Stanford Bunny with its own digital stereolithography information embedded into its DNA. This self-replicating property may be used to perfectly create six generations of the same object, where each new generation obtains its DNA memory from the previous generation without additional DNA synthesis. DoT can be used for multiple applications such as steganography, where data is concealed in common objects, development of anti-counterfeit material, supply chain tracking, and the advancement of research in self-replicating machines.

The world's data is growing at exponential rates. However, attempts to further miniaturize traditional storage architectures, such as hard-drives and magnetic tapes, are difficult. These devices have reached their physical limitations and cannot keep pace with digital storage requirements. Due to these challenges, DNA molecules may be used as an architecture for long term cold storage. DNA storage may reach three orders of magnitude higher physical densities than traditional devices and can have a half-life of thousands of years.

In addition to having exceptional density and endurance, DNA storage is virtually the only storage architecture that can take any shape. This stands in stark contrast to traditional storage architectures such as tapes or hard-drives, where the actual shape of device is often critical to its functionality. Studies of DNA storage have largely overlooked the virtue of the absence of shape constraints. However, this property allows the realization of new storage architectures beyond today's conventional designs.

The present disclosure provides for a storage architecture, dubbed the DNA of Things (DoT), in which DNA molecules are fused to a functional material to create objects with immutable memory. According to embodiments of the present disclosure, the DoT architecture starts with encoding the data in DNA molecules in a manner that is robust to errors. Due to the low density of the DNA molecules in the embedding material, it is possible that some DNA fragments will not be recovered from the object, creating fragment dropouts. To ameliorate that, various data encoding schemes may be employed, such as fountain coding. One example of a suitable encoding scheme is DNA Fountain, described in U. S. Patent Application publication No. US2019/0020353A1. This scheme provides high flexibility in setting virtually any redundancy level that can correct dropout errors and perfectly retrieves data from minute quantities of material.

DNA is not soluble in many materials, so mixing is not possible. Where mixing DNA is possible, simply mixing the DNA with the functional material results in quickly degraded DNA due to hydrolysis stress and elevated temperatures during the preparation of the mixture. To mitigate that, the DoT architecture first encapsulates the DNA in silica nanoparticles, resulting with silica particle encapsulated DNA (SPED). The SPED sequesters the DNA molecules, prolonging their half-life, and facilitating the mixing of DNA with the embedding material. The encapsulation enables mixing processes, as encapsulation materials (e.g., silica nanoparticles) allow better miscibility in a wide range of materials. In addition, chemical/thermal stability is introduced due to the encapsulation.

Referring to **Fig. 1,** a plot of the robustness of encapsulated DNA and free DNA against various external factors according to embodiments of the present disclosure is shown. The chemical and thermal stability of encapsulated DNA compared to non-encapsulated DNA is indicated by the fraction of DNA recovered under various conditions. In various experiments, for the thermal stability assessment, solid state DNA and SPED encapsulated DNA were stored at 60°C for one week at 50% relative humidity. For stability against aggressive chemical species, DNA and encapsulated DNA were treated with bleach and reactive oxygen species (ROS) in aqueous solution. * denotes values < 10'5. Error bars represent standard errors of experimental triplicates. A wide range of embedding materials can be used for the DoT architecture. In various embodiments, the SPED beads are embedded in polycaprolactone (PCL). PCL is a biodegradable thermoplastic polyester that offers low melting temperature and high solubility in various organic solvents, which make it an ideal material for blending and printing under mild conditions. To prepare 3D printing filaments, the SPED capsules may be mixed with dissolved PCL and the mixture may be extruded into 2.85 mm filaments that are compatible with desktop 3D printers.

Referring to **Fig. 2,** a visualization of the DNA of things (DoT) process according to embodiments of the present disclosure is shown. The digital file is encoded into a DNA oligo library using DNA Fountain encoding. The synthesized library is encapsulated by a sol-gel synthesis method into small glass particles and blended into PCL, which is extruded into standard 3D-printing filament. The object, as defined by the initial digital file, is printed with the DNA comprising filament. The DNA library can be extracted from any part of the printed object and amplified by PCR. By sequencing the DNA and decoding the DNA Fountain, the original .stl file can be retrieved to 3D print new objects.

To empirically test data storage using the DoT architecture, 3D object **201** may be created that holds its own digital information within its embedded DNA. This configuration is similar to parthenogenesis in biological systems in which the instructions of making objects reside within the matter itself and transmitted (as shown in the visual comparison of **Fig. 3.**).

Referring to **Fig. 3,** a visual comparison of the pathogenesis (left) and DoT process according to embodiments of the present disclosure (right) is shown. The crustacean p. *Virginalis* [Phenotype] can reproduce asexually, generating identical (female) clones [New generation of identical clones], and uses DNA [Genotype] to transmit information from one generation to the next. In the same manner, clones of the DoT Stanford Bunny [New generation of identical clones]can be generated from the synthetic DNA [Genotype] within the paternal object [Phenotype]. Apparently, biological system comprises all processes for self-replication. The replication of the DoT object according to this invention requires external devices, such as DNA sequencer, thermal cycler, extruder, 3D printer, for successful cloning.

In various experiments, the Stanford Bunny, which is a common computer graphics 3D test model, was selected as the object. First, the binary stereolithography (stl) file of the bunny was compressed from 100 kB to 45 kB. Next, DNA Fountain was used to encode the file in 12,000 DNA oligos, which is the maximal number of oligos produced by a single CustomArray chip. With this number of oligos compared to the file size, DNA Fountain encoding yields a redundancy level of 5.2x, a dropout of even 80% of the DNA oligos can be tolerated while still allowing the correct decoding of the file.

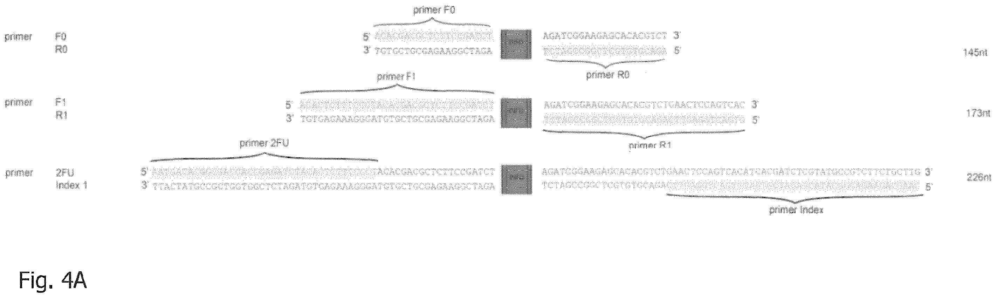

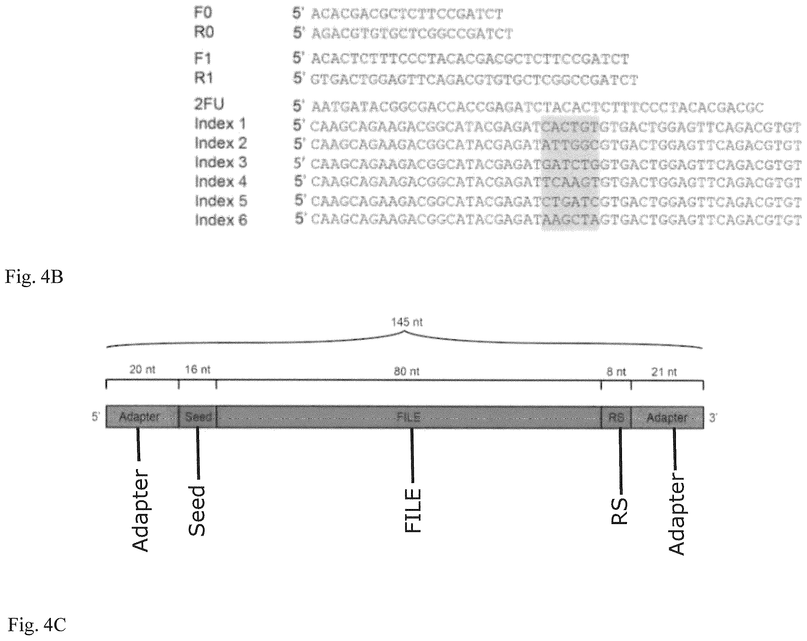

Referring to **Figs. 4A-C,** schematics of a sequence design for the DoT process according to experiments of embodiments of the present disclosure are shown. **Fig. 4A** shows a primer design of sequencing adapters as used in experiments of the present disclosure. **Fig. 4B** shows a list of primer and index sequences used in experiments of the present disclosure. **Fig. 4C** shows oligo architecture for each of 12,000 features Oligonucleotide sequences as used in various experiments of the present disclosure.

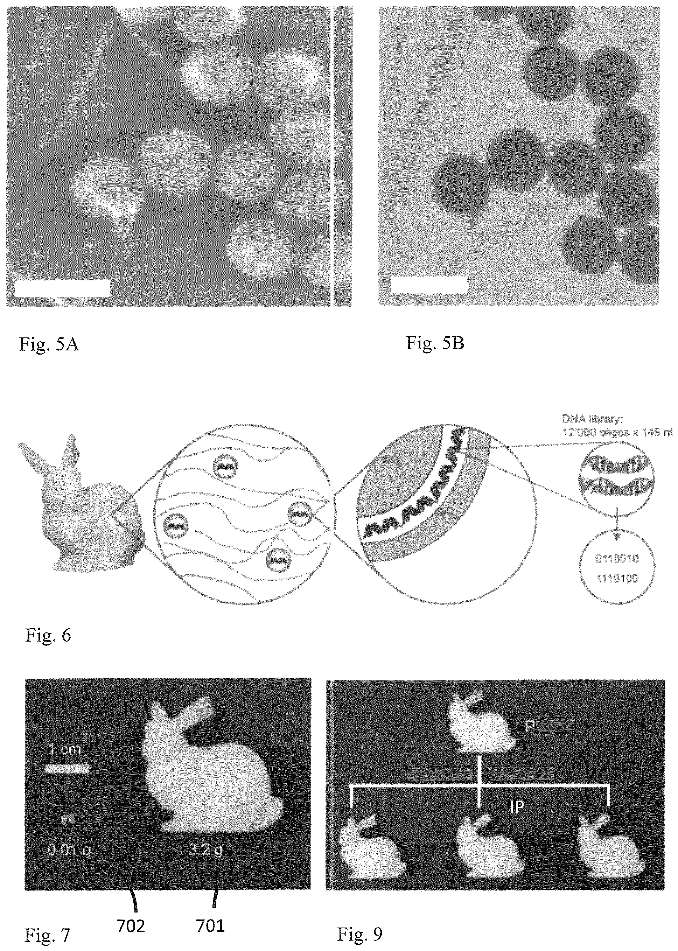

Referring to **Figs. 5A-B,** electron microscopy images of silica particle encapsulated DNA (SPED) according to embodiments of the present disclosure are shown. **Fig. 5A** and **Fig. 5B** show scanning and scanning transmission electron microscopy images of the SPEDs, respectively, which are about 160nm large particles comprising each ca. 60 DNA strands (DNA not visible). The scale bar is 200nm.

Referring to **Fig. 6**, a visualization of the hierarchical architecture of a **3D** printed bunny containing information within its DNA according to embodiments of the present disclosure is shown. The bunny contains its building instructions within its DNA.

In various experiments, the length of the oligos was 145nt, consisting of 104nt of payload and 41nt for PCR annealing sites (as shown in **Figs. 4A-C).** Next, the PCR-amplified oligos were loaded into SPED beads (as shown in **Figs. 5A-B)** and embedded in a PCL filament. The filament contained SPED beads in a concentration of lOO mg/kg (=100ppm), which does not create any detectable changes to the mechanical properties, weight, or color of the filament. The DNA loading within SPED was 2mg of DNA per gram of SPED beads, which corresponds to a DNA concentration of 0.2mg/kg (=0.2ppm) of PCL filament (as shown in **Fig** 6), well below the concentration of DNA in biological organism compared to their body weight (~1OOO ppm in *E.coli* ). Finally, the Stanford Bunny was 3D printed using the same file stored in the DNA containing PCL filament.

Results of experiments according to the present disclosure show that the data can be perfectly and rapidly retrieved from the 3D object by consuming a minute quantity of material using a portable sequencer.

Referring to **Fig. 7**, a visualization of a step in a DoT process according to embodiments

of the present disclosure is shown. In various experiments, ~10 mg of the printed PCL was clipped from the ear of bunny **701,** which is 0.3% of the total material of bunny **701** that weighted 3.2 g . The lOmg 702 of the parent 3D printed object 701 was sufficient to recover enough DNA with accurate building instructions (.stl file) to generate identical progeny. Next, the SPED beads from the embedding PCL were released using tetrahydrofuran (THF), the DNA from the SPED beads was extracted using buffered oxide etch (BOE), and the library was purified using a standard PCR cleaning kit.

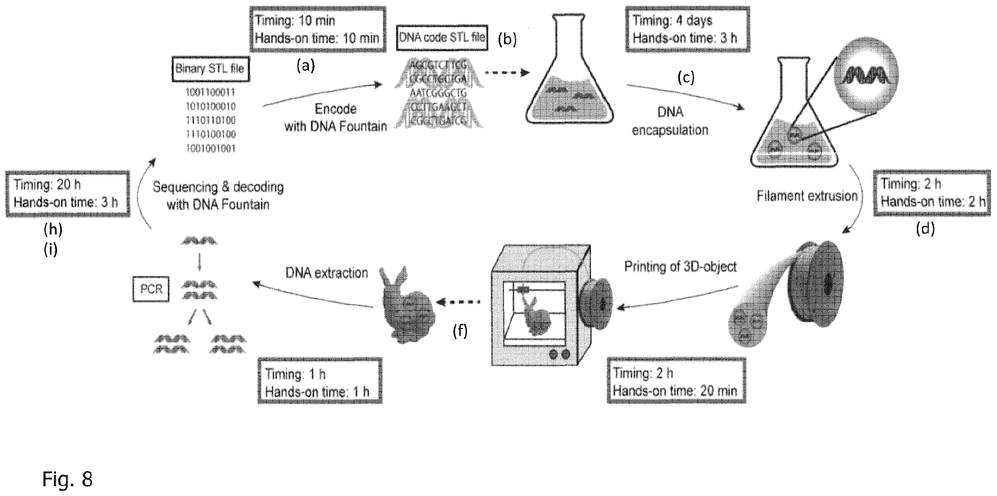

Referring to **Fig. 8,** a visualization of the DoT process according to embodiments of the present disclosure is shown. **Fig 8** shows handling and overall process timing for the individual steps in the formation and reading of the DoT objects. In various experiments, the recovered DNA library weighed 25pg in 50pL volume, corres ponding to about 14,000 copies of the encoded file, including the 5.4x redundancy. This entire process took 4 hours end-to-end. IpL of the recovered DNA, equivalent to 1/50 of the recovered DNA, was then amplified using 10 PCR cycles and the library was sequenced using an iSeq, a portable Illumina sequencer. This process took 17 hours and yielded 1,046,118 reads. Finally, the data was processed using the DNA Fountain decoder and the stored .stl file was perfectly retrieved despite missing 5.9% of the original oligos and sequencing errors, which took a few minutes on a standard laptop (as shown in **Fig. 8**).

Multi-round replication experiments were conducted using the DoT architecture. In the first replication round, the PCR-amplified DNA from the parent Stanford Bunny (P) was fused to a nascent PCL filament using the DoT procedure. Referring to **Fig. 9**, a visualization of a step in a DoT process according to embodiments of the present disclosure is shown. In **Fig. 9**, the initial "parent" bunny and three "children" printed with the DNA instructions embedded in the parent DNA are shown. The progeny also hold DNA instructions using the original DNA library. In various experiments, three offspring 3D structures (FI) were created using the PCL filament and the retrieved .stl file (as shown in **Fig. 9**). Subsequently, about **10** mg was clipped from one of the FI, the DNA was extracted, and the library was sequenced with iSeq to retrieve the .stl file, and 3D object was printed as the next generation. The same procedure was repeated for five generations, where in each generation a new PCL filament was created by fusing the PCR amplified DNA molecules of the previous experiment.

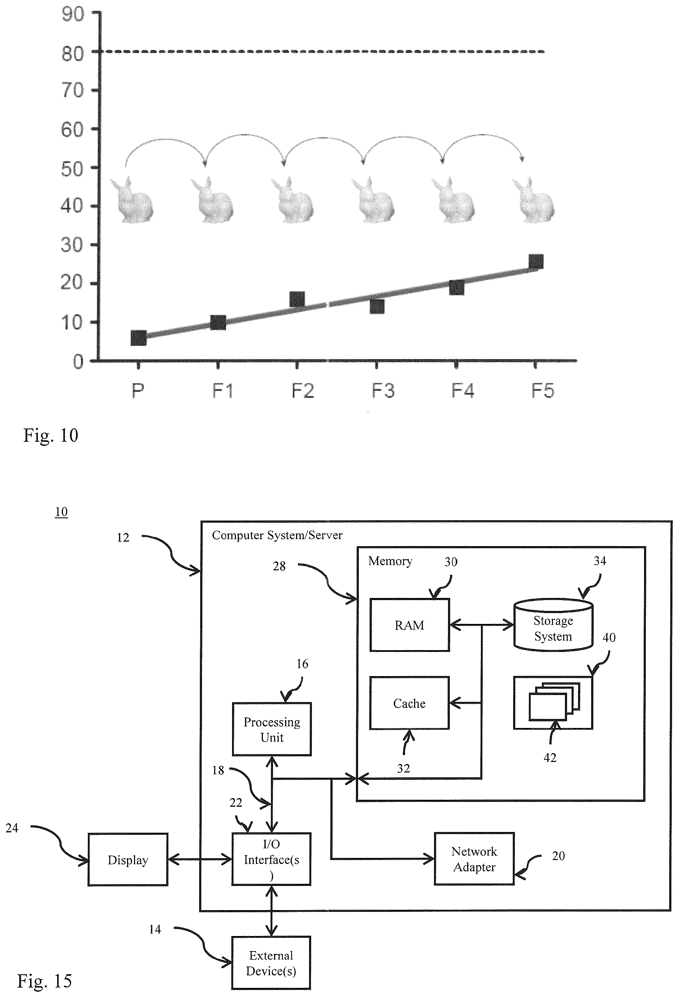

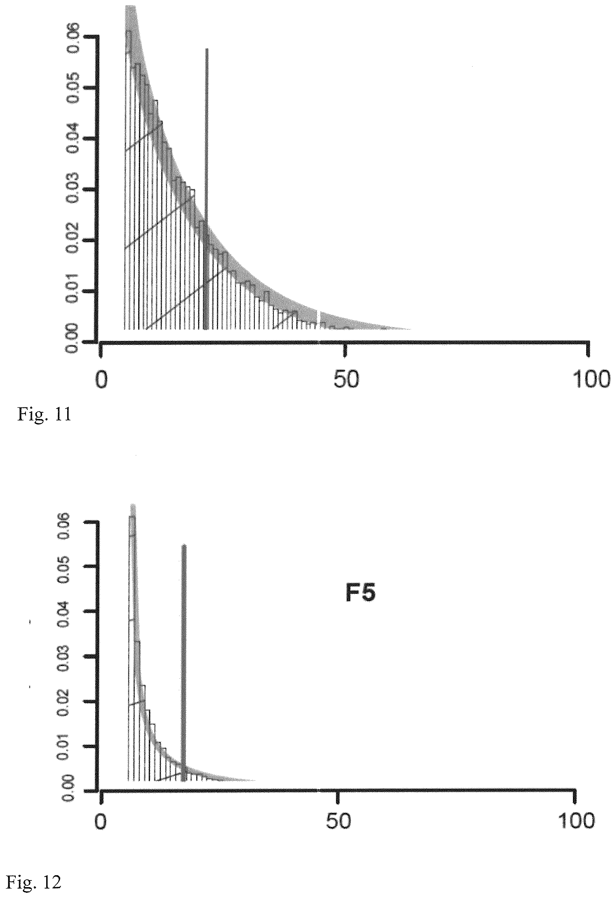

Referring to **Fig. 10,** a plot of the file corruption rate for various generations of a DoT object according to embodiments of the present disclosure is shown. The file was perfectly retrieved from all five generations of progeny. However, in nearly each replication round, an increase in the fraction of drop-out molecules was recorded from a level of 5.9% for P to a level of 25.6% in F5. For every new generation, the file corruption rate increased, yet for the 6 experimentally implemented generations, it remained well below the file corruption rate that the Fountain error correction code allowed. To better demonstrate the effect of replication on the library composition, w the number of correct sequence reads per oligo was modeled using a negative binomial distribution. **Figs. 11** and **12** show plots of the oligo coverage frequency for the parent (P) and 5th descendant (F5) generations, respectively, of a DoT object according to embodiments of the present disclosure. **Figs. 11** and **12** show an escalation in the negative binomial over-dispersion parameter (1/size) from 1.0 in P **(Fig. 11)** to 2.27 in F5 **(Fig. 12).** This increase in over-dispersion means that the oligos become less equally represented with successive generations, which may be ascribed to the added thermal stress during polymer extrusion/printing and increasing numbers of PCR rounds. Yet, the DNA Fountain strategy easily dealt with these issues and retrieved the file correctly for all generations using the same procedure.

The economy of the DoT architecture is consistent with mass production of goods with memory at negligible per-unit costs. Each replication consumes only 0.3% from each bunny and yields sufficient DNA material to create 29 offspring bunnies. Therefore, even if the number of replications are restricted to five generations, it is theoretically possible to create at least $(29/0.003)^6 = 8.15 \times l 023$ of bunnies without resynthesizing the DNA library. Moreover, if the linear trend of increase in drop-out continues with each progeny (as seen in **Fig. 10**), generation F15 will be well within the maximal tolerance to dropouts of 80%. Thus, it is possible to create $(29/0.003)^{15}$ _ 6xl 059 copies without resynthesizing the DNA library, which exceeds the amount atoms available on earth to create these bunnies, and $29^{15}$ 86x1020 bunnies if only the tip of the ear is allowed to be clipped, and not any other part of the bunny. Thus, despite the relatively high upfront costs to synthesize the file for the first time, the per-unit costs of storing data is negligible, as mass production requires only one synthesis event.

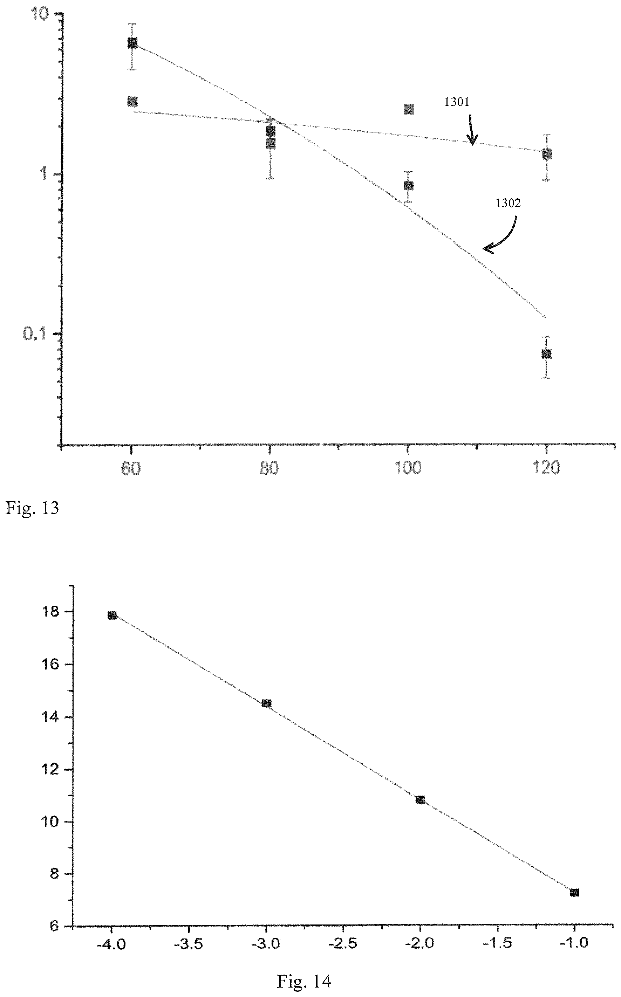

The DoT architecture is compatible with a wide range of embedding materials beyond PCL. For such compatability, the DNA library survives the melting temperature of the embedding material and the material formation chemistry, which may include radical oxygen species (RoS) or other types of aggressive chemical reactions. Referring to Fig. 13, a plot of the thermal stability of an encapsulated DNA file for different processes and temperatures according to embodiments of the present disclosure is shown. The thermal stability of an encapsulated DNA file in PCL filament for different 3D printing (1301) and filament extrusion (1302) process temperatures is shown. Error bars represent standard errors of experimental triplicates. Lines 1302 and 1301 represent a nonlinear regression for the filament extrusion and 3D printing processes, respectively, according to Arrhenius law for a first order decay reaction (see below).

To test the robustness of the present architecture, qPCR was used to measure the amount of DNA retrieved from the SPED beads in PCL using various extrusion and 3D printing temperatures. The amount of recovered DNA followed Arrhenius expression trends. For the extrusion process, with each 30C increase in temperature, there is a drop of approximately one order of magnitude in the recovered DNA. On the other hand, only a relatively slow decline in the recovered DNA was observed with the increase of the 3D printing temperature, as this process is much faster than extrusion and therefore exposes the library to high heat only for a short amount of time. It is also possible to use elevated temperatures for short cycle times during polymer processing while suffering some losses of DNA, which may be compensated by loading more DNA into the polymer preforms. Such temperatures allow the use of DoT for selected polymer injection molding processes, and pre-impregnated carbon fiber composite autoclaving.

The robustness of the library to reactive oxygen species (RoS) was evaluated using the Fenton-like copper reaction and bleach oxidation. The encapsulation of the DNA is sufficient to protect against these extreme conditions without any detectable change in the amount of DNA. As such, this robustness to RoS and oxidation agents enables DoT to be embedded in materials manufactured by radical polymerization, such as polystyrene, polyacrylates and acrylamides. Thus, a wide range of materials can be used to embed the DNA library.

The DoT architecture is useful in applications where blueprint level information is required to be accessible. For example, in the field of 3D medical or dental implants, each structure is unique and customized for the price anatomical structure of the patient. As SPED beads are non-toxic, DoT allows the storage of the design of the implant together with other medical background information in the implant, offering a long-range back-up alternative to traditional electronic medical records (EMR) that are usually required to be kept only for 5-10 years. Similarly, DoT is applicable for building materials, pharmaceutical products, and electronic components. All of these are areas that require product control information, which is not available at the point of usage. Having relevant information directly integrated via DoT into the product in an invisible, distributed, and non-removable manner allows for tighter and better controlled product quality measures. This requires fast DoT retrieval protocols and ubiquitous DNA sequencers.

The DoT architecture is also applicable in information steganography. For example, information steganography may be provided by hiding data in DNA as a form of microdots. The DoT architecture extends this concept and enables to turn a wide range of everyday objects, from a keychain to a bottle lid, into concealed storage devices that can secretly carry data. An adversary trying to intercept the data faces multiple barriers: first, the SPED beads do not change the properties of the material, thus, the adversary will have to test multiple objects to reveal the concealing object. Second, the DNA is sequestered in the SPED, so regular DNA sensing techniques such as UV light are not helpful. Third, even if the library is recovered, the adversary will have to know the annealing sites to PCR amplify the message. Accordingly, DoT is highly resistant to malicious interception.

Another application of DoT is in the area of self-replicating devices. One DoT library has sufficient replicating capacity to produce storage material for virtually unlimited supply of objects. This simplifies blueprint transmission in self-replicating devices. With traditional storage architectures, the self-replicating device needs to *ab-initio* build a data storage compartment (*e.g.,* hard-drive), which requires a large number of components and environmentally controlled conditions. With DoT, a self-replicating device can transmit its blueprint via a PCR procedure, which can be achieved with a limited number of components. It will be appreciated that the DoT architecture requires a small variety of components. More generally, increasing data flow leads to increased dependency on delocalized data storage and large-scale data centers. The DoT approach provides a powerful alternative for localized and off-the-grid storage technology, capable of storing sensitive and precious data for multiple generations. It may be used as well for integrating fabrication plans and conditions into complex products such as houses or art installations, as a way of preserving information for later generations.

In an exemplary experiment, DNA Decoding was achieved as follows. The original binary stereolithography (stl) file was downloaded from graphics.stanford.edu and was reduced in resolution from 22 MB to 100 kB by reducing the vertex count of the mesh with Blender 2.79b (Blender Foundation). Next, the file was compressed using gzip and the data was encoded using the DNA Fountain software with the following parameters: -maxhomopolymer 3 -gc 0.05 -rs 2 -size 20 -delta 0.001 -c dist 0.025nt of 50%±5%, 2 bytes of Reed-Solomon error correcting code, 20 bytes of data in each oligo, and Robust Soliton Distribution parameters of c=0.025 and 5=0.001, and 12,000 oligos, respectively.

In this example, the library synthesis was achieved as follows. The DNA Fountain output was a library with 12,000 oligos of length 104nt. The following PCR annealing sequences were added to flank each oligo: 5'-ACACGACGCTC TTCCGATCT-3' (20nt) and 5'- AGATCGGAAGAGCAC ACGTCT-31 (21nt), so the resulting oligos were of length 145nt. The oligos were synthesized with CustomArray Inc. (Bothell, WA). 47 ng from the synthesized library was PCR amplified using the following primers: 5-'ACACGACGCTC TTCCGATCT-3' and 5'-AGACGTGTGCTCTTCCGATCT-3' and KAPA SYBR® FAST qPCR Master Mix (KAPA Biosystems) using the following cycling parameters: 95°C for 15s, 54°C for 30s, 72°C for 30s for 30 cycles.

Referring to **Fig. 14,** a plot of the qPCR cycle threshold for various SPED concentrations according to embodiments of the present disclosure is shown. In this example, 12.2pg of DNA was loaded onto 1.75 mg silica nanoparticles, functionalized with N-trimethoxysilylpropyl-N,N,N-trimethylammonium chloride (TMAPS) and subsequently coated with an additional silica layer by adding tetraethoxysilane (TEOS).

In this example, the incorporation into polymer and 3D-printing was done as follows. 2 mg of SPED were centrifuged at 15,000 rpm for 3 min and the supernatant was removed. The particles were then washed and centrifuged 2x in 2 mL Eppendorf tubes with 2 mL acetone (>99.8%, ProLabo). The particles were then suspended in 2 mL THF (VWR >99.5%) and added to THF-dissolved PCL (MakerBot, 20 wt% PCL in THF). To distribute the particles in the polymer, the suspension was mixed with a high speed mixer (SpeedMixer DAC 150 FVZ). After solvent evaporation under a fume hood, the solid polymer was extruded (Filastruder) at 60°C to a 2.85 mm filament. Objects were then printed with a commercial 3D-printer (Ultimaker 2+, Ultimaker) modified with a geared direct drive extruder and a V6 hotend (E3D). The parts were printed onto a heated build-plate (45°C) that was coated with a layer of adhesive spray (3DLac) at a speed of 20 mm/s and forced convection was applied to cool the extrudate. The Stanford bunny was printed with a nozzle temperature of 100°C. An open-source FDM sheer (Cura, Ultimaker) was used to generate the print paths.

In this example, sequencing and product cycle generation was done as follows. ~10 mg was clipped from the bunny ear using a scalpel. This piece was dissolved in 250 pL THF (VWR International) before 10 pL of buffered oxide etch (BOE) solution were added. The BOE was added in order to release the DNA molecules from the SPED beads. The DNA was then purified by PCR Purification Kit (Qiagen) and PCR using the same qPCR protocol as for the library synthesis. The different DNA samples from each generation were pooled and diluted to 1 nM and mixed with an additional 2% spike of PhiX, added for base diversity. Finally, the DNA pool was sequenced with Illumina iSeqlOO using the 150nt paired-end protocol. A detailed procedure on sequencing preparation is provided below.

In this example, decoding was done as follows: For each generation, the paired-end sequencing data was stitched together using Pear. Sequence reads whose length was not equal to original design length 104nt were removed using awk. Next, a decoding strategy similar to the DNA Fountain scheme was used. Reads were first collapsed and sorted based on their appearance from the most abundant to the least abundant, breaking ties lexicographically. Then, the file was decoded back using the DNA Fountain that employs a message passing algorithm scheme. The input SHA256 of the compressed stl file (314d00155dcbc259683cfc02fbe9edbbf205a978f573569c79ab2dlb 5e6bdc53) was compared to the SHA256 of the retrieved file of each generation. In all cases, the SHA256 signatures were identical. Subsequently, the extracted file was printed as described in the previous section. Fitting the negative binomial distribution was done in R using the tdistr command.

In this example, thermal and chemical stability assays were done as follows: DNA- infused PCL was extruded and 3D-printed at temperatures ranging from 60°C to 120°C. Assessing long-term stability, dried pure and SPED DNA libraries were stored at 60°C for 10 days and then re-suspended and quantified by qPCR. Chemical stability was tested by a radical treatment stability assay, where a combination of ascorbic acid, H2O2, and CuCh produce reactive oxidative species and test the protective properties of SPED (see below for details).

Further details of these experimental steps are provided below.

DNA synthesis

The 12,000 DNA sequences were synthesized by CustomArray Inc. (Bothell, WA) on a 12K chip. The DNA was received in ssDNA form at a concentration of 46.93 ng/pL. The oligo pool was first amplified by PCR in order to dilute any fragmented features and to have sufficient material for silica encapsulation. For this, the DNA was diluted 1:46 and a standard PCR was performed with the following reaction mixture in each 96-well: 10 pL master mix (Roche, Lightcycler 480 SYBR Green I Master Mix), 8 pL of MilliQ grade water, 1 pL of primer mix (primers F0 and R0 (Microsynth AG), each at lOpM) and 1 pL diluted DNA pool. Cycling parameters were 95°C for 15s, 54°C for 30s, 72°C for 30s for 30 cycles. Once finished, 4 wells were pooled together (80pL) and purified by QIAGEN QIAquick PCR Purification Kit with 150pL elution buffer used per column. This yielded 1050pL of 10ng/pL DNA solution.

Particle synthesis

The synthesis of SPED was achieved as follows: 50 mg silica nanoparticles (microparticles GmbH, 0 l5Onm) in 1 mL ethanol were surface functionalized with 10 pL N-trimethoxysilylpropyl-N,N,N - trimethylammonium chloride (TMAPS, 50% MeOH, ABCR GmbH) for 12 h. The surface potential of the functionalized silica particles was measured to be +47 mV by a zeta potential analyzer (Zetasizer Nano, Malvern). To produce 1 batch of 1.75 SPED, 35pL of particles (50 mg/mL) were added to 1050 pL of double stranded DNA (10-12 ng/pL). The amount of unbound DNA was determined by NanoDrop 2000c Spectrophotometer (0.7-1 ng/pL). On top of the DNA layer, silica was grown through Stober synthesis. 0.5 pL of TMAPS and 0.5 pL of tetraethyl orthosilicate (TEOS, >90%, Aldrich) were added before shaking for 5 h. Then, 4 pL TEOS was added and the batch was shaken for 4 days. The DNA loading was determined by QEIBIT Fluorometer DNA assay (ThermoFisher Scientific). To 100 pL of a particle suspension with known concentration, 3 pL of BOE were added. The DNA concentration of 5 pL of this suspension was then measured by QEIBIT Fluorometer (0.744 ng/pL DNA in 0.35 g/L particle suspension, 0.2wt% DNA loading on particles).

Sequencing

Six DNA libraries from F0 to F5 were sequenced. The DNA was isolated by clipping 10 mg of PCL and dissolved in 250 pL THF and 10 pL BOE. The DNA was then purified by PCR Purification Kit (Qiagen). All libraries were first diluted to equal concentrations. This was done so that each sample went through the same number of PCR cycles and had the same amount of amplification errors introduced. The DNA was then amplified with FI and R1 primer with the following mix: 10 pL master mix, 8 pL of MilliQ water, 1 pL primer stock (FI and R1 (Microsynth, Switzerland), 10 pM each), 1 pL DNA sample). The cycling parameters were as follows: 95°C for 15 s, 54°C for 30 s, 72°C for 30 s, 25 cycles. The DNA was extracted by gel electrophoresis. The band slightly below 200nt was cut out and purified with QIAquick Gel Extraction Kit (QIAGEN, Germany). Each library was indexed by a second PCR with the general forward primer 2FU and an indexed reverse primer. The qPCR mix with the following mix: 10 pL master mix, 8 pL of MilliQ water, 1 pL primer stock (2FE1 and indexed primer, 10 pM each), 1 pL of 1:10 diluted DNA sample). The cycling parameters were as follows: 95°C for 15 s, 53°C for 30 s, 72°C for 30 s, 10 cycles. Each qPCR product was purified by gel electrophoresis and the DNA concentration was measured by Qubit assay. All samples were pooled to a 1 nM library and then further diluted to 20 pM. Prior to sequencing 2xl 50bp on the iSeq 100 (illumina, San Diego CA), 3% of PhiX were added to ensure call diversity

Comparison to biology

The DoT Stanford Bunny demonstrates remarkable similarities to biological organisms by carrying its 'blueprint' within its DNA. In multicellular organisms, the genomic DNA is distributed throughout the biological systems in small compartments. As such, the replication of the DoT object can be compared to asexual biological reproduction such as budding and parthenogenesis. Multicellular budding as observed in hydra comes from the fact that any small part of the original system can be utilized to generate daughter clones. The comparison with parthenogenesis, as shown in **Fig. 3**, emphasizes that a reproduction machinery is required to generate identical clones. Parthenogenesis, as observed in the invasive crayfish *speciesp. Virginalis,* but also reported for many other crustaceans, insects, squamata and several other animal phyla, is a form of asexual reproduction, where offspring develop directly from a unfertilized egg. In biology, the reproduction machinery of an adult, therefore, requires the formation of an egg cell containing a full genome copy, and the outgrowth of offspring from this egg via cell division and differentiation facilitated by DNA transcription and translation. In comparison, the presented DoT system reproduction machinery requires a DNA sequencer to read the genome, a computer to translate the DNA sequence to a .stl file and a 3D printer to physically generate the offspring from raw material. In order to ensure that the offspring also contains the full genome, the original DNA is amplified via PCR and mixed into the raw material.

Mass of copy

An oligo consists of 145 base pairs and the full file is stored on 12'000 oligos. Taking 650 g/mol as the average weight per base pair, the minimum amount of DNA needed for one full copy can be determined: ((650 g/mol) /6.022e23) *145nt*12'000 = 1.88e-15 g DNA per copy.

DNA quantification

Quantitative PCR was used to quantify DNA content and degradation. A dilution series from E-l to E-4 g/L of SPED was measured to serve as a standard curve. To each dilution step, 10 pL BOE was added to 10 pL sample analogous to 10 mg of PCL. The DNA was then purified by QIAGEN QIAquick PCR Purification Kit with 50 pL elution buffer used per column. The reported values are averaged over triplicates.

Thermal stability assays

DNA-infused PCL was extruded and 3D-printed at temperatures ranging from 60°C to 120°C. Of this extruded or printed material, 10 mg were clipped and dissolved in 250 pL THF, followed by purification with QIAGEN QIAquick PCR Purification Kit with 50 pL elution buffer used per column. Assessing long-term stability, 0.492 pg DNA and 3.5 pg SPED were dried in a vacuum centrifuge (Eppendorf Concentrator plus) and stored at 60°C and 50 %RH for 10 days and then re-suspended in 100 pL and quantified by qPCR (10 pL master mix (Roche, Lightcycler 480 SYBR Green I Master Mix), 8 pL of MilliQ grade water, 1 pL of primer mix (primers FO and RO (Microsynth AG), each at 10 pM), 1 pL diluted DNA pool, Cycling parameters were 95°C for 15 s, 54°C for 30 s, 72°C for 30 s for 30 cycles).

Table 1

|  | Encapsulated (Cycle threshold) | Non-encapsulated (Cycle threshold) |
|---|---|---|
| Control 5°C | 6.98±0.03 | 11.47±0.08 |
| 60°C | 9.158±0.001 | 18.922±0.004 |

Chemical stability assays

Encapsulated and non-encapsulated DNA were subjected to radical treatment stability assay, where a combination of ascorbic acid, H2O2, and CuCb produce reactive oxidative species and test the protective properties of SPED towards DNA oxidation. 5 pL of DNA/encapsulated DNA were added to 2.5 pL L-ascorbic acid (20 mM), 12.5 pL H2O2 (20 mM) and 17.5 pL CuCh (500 pM). After 10 minutes, the reaction was quenched by adding 17.5 pL of 100 mM EDTA and 20 pL of BOE. To measure the total amount of DNA, 50 pL of water and 20 pL BOE were added to 5 pL of DNA/encapsulated DNA. The DNA concentration of treated and untreated samples was measured by QUBIT assay. All reported values are averaged over quintuplets.

Table 2

|  | Encapsulated (ng/pL) | Non-encapsulated (ng/pL) |
|---|---|---|
| Control | $0.289 \pm 0.003$ | $0.288 \pm 0.002$ |
| ROS assay | $0.29 \pm 0.06$ | $0.03 \pm 0.02$ |

The DNA stability was also evaluated by subjecting it to household bleach. A bleach stock was prepared with 8.57 mL NaClO (14% activity), 1.164 mL NaOH 10 M and 30.266 mL H2O. 143 pL of 100-fold diluted bleach stock was added to 143 pL of DNA (10 ng/ml) or SPED suspension (0.1 pg/mL) and incubated for 10 min. The reaction was quenched by adding 5 pL of thiosulfate (1.46 M). The unprotected DNA is directly measured by qPCR whereas for the SPED samples, 10 pL of BOE were added and then purified by QIAGEN QIAquick PCR Purification Kit with 50 pL elution buffer. The samples were quantified by qPCR (10 pL master mix (Roche, Lightcycler 480 SYBR Green I Master Mix), 8 pL of MilliQ grade water, 1 pL of primer mix (primers F0 and R0 (Microsynth AG), each at 10 pM), 1 pL diluted DNA pool, Cycling parameters were 95°C for 15 s, 54°C for 30 s, 72°C for 30 s for 30 cycles). All reported values are averaged over triplicates.

Table 3

|  | Encapsulated (Cycle threshold) | Non-encapsulated (Cycle threshold) |
|---|---|---|
| Control | $4.7 \pm 0.1$ | $9.36 \pm 0.04$ |
| Bleach assay | $5.26 \pm 0.05$ | $29.6 \pm 0.9$ |

Arrhenius fitting function for Fig 3

For DNA decay, a first order chemical degradation reaction can be assumed, where the relative concentration (A/Ao) depends on the reaction rate (k) and reaction time (t),

$$\frac{A}{A_0} = e^{-k\,t}.$$

*Equation 1*

The temperature (T) dependence of the reaction rate is given by the Arrhenius law, involving a pre-exponential factor ko, the activation energy (Ea) and the gas constant (R):

$$= k_0 e^{-\frac{E_a}{RT}}$$

*Equation 2*

The final equation for the relative concentration as a function of reaction temperature is obtained by inserting the Arrhenius law into the rate equation:

$$\frac{A}{A_0} = e^{-k_0 e^{-\frac{E_a}{RT}} t}$$

*Equation 3*

Nonlinear curve regression was performed for A/Ao vs. T assuming constant ko, Ea and reaction time t.

Half life calculation at room temperature

Again, first order kinetics for DNA degradation is assumed with the relative DNA concentration (A/Ao), the reaction rate (k) and time (t).

$$\frac{A}{A_0} = e^{-k\,t}$$

*Equation 4*

After storing the library at 60°C for 10 days, 65% of the initial DNA was recovered, allowing the calculation k at T=60°C. By using the Arrhenius law and an activation energy of 158 kJ/mol, k0 can be calculated with the pre-exponential factor (ko), activation energy (Ea) and the gas constant (R).

$$k = k_0 e^{-\frac{E_a}{RT}}$$

*Equation 5*

Assuming a storage temperature of 20°C, the rate constant with Arrhenius law can be calculated. Finally the half life of encapsulated DNA at t=20°C is found by:

$$t_{\frac{1}{2}} = \frac{\ln(2)}{k(T)}$$

*Equation 6*

Various examples provided herein use DNA or RNA. However, it will be appreciated that any sequence-derived polymer may be used as described herein. In general, a sequence-controlled polymer is a macromolecule in which the sequence of monomers is controlled. A sequence-controlled polymer can be uniform (with dispersity equal to one) or non-uniform (with dispersity greater than one). An alternating copolymer synthesized by radical polymerization is a sequence-controlled polymer, even if it is non-uniform polymer. A biopolymer (for example a protein) with a perfectly-defined primary structure is also a sequence-controlled polymer. In the case of uniform macromolecules, the term sequence-defined polymer can also be used. The composition of sequence-controlled polymers can be defined via chemical synthetic methods, such as multicomponent reactions or click reactions. DNA, RNA, and proteins are examples of sequence-controlled polymers.

Referring now to **Fig. 15,** a schematic of an example of a computing node is shown. Computing node **10** is only one example of a suitable computing node and is not intended to suggest any limitation as to the scope of use or functionality of embodiments described herein. Regardless, computing node **10** is capable of being implemented and/or performing any of the functionality set forth hereinabove.

In computing node **10** there is a computer system/server **12,** which is operational with numerous other general purpose or special purpose computing system environments or configurations. Examples of well-known computing systems, environments, and/or configurations that may be suitable for use with computer system/server **12** include, but are not limited to, personal computer systems, server computer systems, thin clients, thick clients, handheld or laptop devices, multiprocessor systems, microprocessor-based systems, set top boxes, programmable consumer electronics, network PCs, minicomputer systems, mainframe computer systems, and distributed cloud computing environments that include any of the above systems or devices, and the like.

Computer system/server **12** may be described in the general context of computer system-executable instructions, such as program modules, being executed by a computer system. Generally, program modules may include routines, programs, objects, components, logic, data structures, and so on that perform particular tasks or implement particular abstract data types. Computer system/server **12** may be practiced in distributed cloud computing environments where tasks are performed by remote processing devices that are linked through a communications network. In a distributed cloud computing environment, program modules may be located in both local and remote computer system storage media including memory storage devices.

As shown in **Fig. 15,** computer system/server **12** in computing node **10** is shown in the form of a general-purpose computing device. The components of computer system/server **12** may include, but are not limited to, one or more processors or processing units **16,** a system memory **28,** and a bus **18** that couples various system components including system memory **28** to processor **16.**

Bus **18** represents one or more of any of several types of bus structures, including a memory bus or memory controller, a peripheral bus, an accelerated graphics port, and a processor or local bus using any of a variety of bus architectures. By way of example, and not limitation, such architectures include Industry Standard Architecture (ISA) bus, Micro Channel Architecture (MCA) bus, Enhanced ISA (EISA) bus, Video Electronics Standards Association (VESA) local bus, Peripheral Component Interconnect (PCI) bus, Peripheral Component Interconnect Express (PCIe), and Advanced Microcontroller Bus Architecture (AMBA).

Computer system/server **12** typically includes a variety of computer system readable media. Such media may be any available media that is accessible by computer system/server **12,** and it includes both volatile and non-volatile media, removable and non-removable media.

System memory **28** can include computer system readable media in the form of volatile memory, such as random access memory (RAM) **30** and/or cache memory 32. Computer system/server **12** may further include other removable/non-removable, volatile/non-volatile computer system storage media. By way of example only, storage system 34 can be provided for reading from and writing to a non-removable, non-volatile magnetic media (not shown and typically called a "hard drive"). Although not shown, a magnetic disk drive for reading from and writing to a removable, non-volatile magnetic disk (e.g., a "floppy disk"), and an optical disk drive for reading from or writing to a removable, non-volatile optical disk such as a CD-ROM, DVD-ROM or other optical media can be provided. In such instances, each can be connected to bus 18 by one or more data media interfaces. As will be further depicted and described below, memory 28 may include at least one program product having a set ( e.g., at least one) of program modules that are configured to carry out the functions of embodiments of the disclosure.

Program/utility **40,** having a set (at least one) of program modules **42,** may be stored in memory **28** by way of example, and not limitation, as well as an operating system, one or more application programs, other program modules, and program data. Each of the operating system, one or more application programs, other program modules, and program data or some combination thereof, may include an implementation of a networking environment. Program modules **42** generally carry out the functions and/or methodologies of embodiments as described herein.

Computer system/server **12** may also communicate with one or more external devices **14** such as a keyboard, a pointing device, a display **24,** etc.; one or more devices that enable a user to interact with computer system/server **12;** and/or any devices (e.g., network card, modem, etc.) that enable computer system/server 12 to communicate with one or more other computing devices. Such communication can occur via Input/Output (I/O) interfaces **22.** Still yet, computer system/server **12** can communicate with one or more networks such as a local area network (LAN), a general wide area network (WAN), and/or a public network ( e.g., the Internet) via network adapter **20.** As depicted, network adapter **20** communicates with the other components of computer system/server **12** via bus **18.** It should be understood that although not shown, other hardware and/or software components could be used in conjunction with computer system/server **12.** Examples, include, but are not limited to: microcode, device drivers, redundant processing units, external disk drive arrays, RAID systems, tape drives, and data archival storage systems, etc.

The present disclosure may be embodied as a system, a method, and/or a computer program product. The computer program product may include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to embodiments of the disclosure. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present disclosure. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

As used herein, particles are homogeneously distributed when approximately the same number of points occurs in any spherical region of a given volume. More particularly, for homogeneously distributed particles in a materials, samples of a material of equivalent volume will have the same number of particles $\pm 10\%$.

In another exemplary application, storage of movie in a transparent PMMA lens was achieved

Storage and retrieval of the Oneg Shabbat video

The video published by UNESCO on YouTube was entitled 'The incredible and moving story of Oneg Shabbat' (https://www.youtube.com/watch?v=yqcLlTbSXUg). It was accessed 14 May 2019 and was reformatted (AAC, H.264) to a resolution of 240pixels at 12 frames per second with 60 kilobase mono audio yielding an mp4 video file of 1.4MB. The digital bitstream was encoded in DNA using DNA Fountain software with the same parameters as for the bunny experiment with two exceptions: first, we let the DNA Fountain software generate 300,000 DNA oligos. Second, we allowed a slightly larger GC content of $50 \pm 20\%$. After appending adapter information, 300,000 DNA sequences (length 145nt) were ordered from Twist Bioscience. The DNA sample ($6\mu g$) was dissolved in $120\mu l$ of Milli-Q grade water to reach a concentration of $50ng\mu l^{-1}$ and stored at -20°C.

Incorporation of Oneg Shabbat library into a PMMA cast

SPED (2mg), which was manufactured from the DNA library using published methods (Paunescu et al. Nat. Protocols. 2012), was centrifuged at 15,000r.p.m. for 3min and the supernatant aqueous solution was removed. The particles were then washed and centrifuged twice in 2-ml Eppendorf tubes with 2ml of acetone (>99.8%, ProL abo). The particles were then suspended in 2ml of acetone by ultrasonication. For casting one pair of PMMA glasses, 0.66mg of particles were added to 7ml of methyl methacrylate (SKresin 1702, S u. K Hock). The suspension was mixed in a 150-ml polypropylene beaker with a high-speed mixer (SpeedMixer DAC 150 FVZ) for 30s at 3,500r.p.m. To initiate the radical polymerization, 70mg of Peroxan (PM25S, S u. K Hock) was added before mixing again for 30s at 3,500r.p.m. with the high-speed mixer. The reaction mixture was left overnight in the beaker, resulting in a transparent PMMA disk. With a scalpel, the form of the glasses lens was cut out of the disk and inserted into the eyeglass frame. For comparing the visibility of PMMA with and without SPED, the same procedure was used to produce a PMMA glass without adding SPED.

Sequencing of Oneg Shabat movie file

10 mg of the PMMA-SPED glass was clipped and dissolved in 500 $\mu L$ acetone (>99.8%, ProLabo). The sample is centrifuged at 15,000 rpm for 3 min and the supernatant is removed. The remaining SPED particles are resuspended in 500 $\mu L$ acetone by vortexing and ultrasound for 1 min. The sample is again centrifuged at 15,000 rpm for 3 min, followed by removing the supernatant. This washing step is repeated once. Finally the particles are suspended in 50 $\mu L$ MilliQ grade water and dissolved by adding 50 $\mu L$ BOE. The DNA was then amplified with F1 and R1 primer with the following mix: 10 $\mu L$ master mix, 8 $\mu L$ of MilliQ water, 1 $\mu L$ primer stock (F1 and R1 (Microsynth, Switzerland), 10 $\mu M$ each), 1 $\mu L$ DNA sample). The cycling parameter were as follows: 95°C for 15 s, 54°C for 30 s, 72°C for 30 s, 25 cycles. The DNA was extracted by gel electrophoresis. The band slightly below 200nt was cut out and purified with QIAquick Gel Extraction Kit (QIAGEN, Germany). The oligo pool was indexed by a second PCR with the general forward primer 2FU and an indexed reverse primer (TrueSeq, GCCAAT). The qPCR mix with the following mix: 10 $\mu L$ master mix, 8 $\mu L$ of MilliQ water, 1 $\mu L$ primer stock (2FU and indexed primer, 10 $\mu M$ each), 1 $\mu L$ of 1:10 diluted DNA sample). The cycling parameter were as follows: 95°C for 15 s, 53°C for 30 s, 72°C for 30 s, 10 cycles. The qPCR mix was purified by gel electrophoresis and the DNA concentration was measured by Qubit assay. The resulting DNA pool was diluted to a 1 nM library for storage. The final sequencing concentration was 20 pM. Prior to sequencing 2x150bp on the iSeq 100 (illumina, San Diego CA), 2% of PhiX were added to ensure call diversity.

Decoding of the Oneg Shabbat video

Very similar steps to those used for decoding the bunny experiment were used to decode the Oneg Shabbat video. The only exception was that during the first round of decoding we noticed that a large number of oligos were sequenced from both their forward and reverse strands. The source of this issue remained unclear but might have stemmed from a parsing error in obtaining the reverse and forward strands from the sequencer. To overcome this issue and to find the correct orientation for each read, we reverse-complemented each sequence read and generated a tandem of two reads sorted lexicographically. For example, if the original read was TTTA then we created the reverse complement, which is AAAT, and then stitched the two reads together in a lexicographical order, resulting in the tandem read AAAT-TTTA. We carried out this process for all reads in the file after the stitching step. Then, we continued with the normal preprocessing steps of collapsing the tandem reads and sorting them based on their appearances. Next, we took each part of the tandem read and evaluated the Reed-Solomon code. We then moved the part that showed zero errors based on the Reed-Solomon code to the decoder. If both parts showed zero errors, it signified that we could not distinguish the orientation and so we discarded the entire tandem read. If none of the parts showed zero errors, we also discarded the entire tandem read. For example, if AAAT showed zero errors based on the Reed-Solomon code and TTTA showed one error, we progressed the AAAT to the decoder and discarded the TTTA. This process resulted in 287,344 unique oligos for decoding. Finally, we decoded the file back using the 287,344 oligos.

The input SHA256 of the video (ef819b4f0fe855 becb53a3a39183d6b8544f24c9c5963f474ec019bbdffe913c) was compared with the SHA256 of the retrieved file, which was identical. It was also possible to play the video using QuickTime player on a standard Mac laptop.

The result shown in the above examples convincingly show how robust and versatile the inventive methods are.

The descriptions of the various embodiments of the present disclosure have been presented for purposes of illustration, but are not intended to be exhaustive or limited to the embodiments disclosed. Many modifications and variations will be apparent to those of ordinary skill in the art. The terminology used herein was chosen to best explain the principles of the embodiments, the practical application or technical improvement over technologies found in the marketplace, or to enable others of ordinary skill in the art to understand the embodiments disclosed herein.

**Claims**

1. A method for storing data in a 3D object using a data containing feedstock, the method comprising the steps of:

   (a) encoding input data into one or more sequence-controlled polymer, wherein encoding the input data comprises applying an error correction code;
   (b) synthesizing the one or more sequence-controlled polymer;
   (c) encapsulating the synthesized one or more sequence-controlled polymer in a plurality of particles;
   (d) embedding the plurality of particles into a feedstock with the plurality of particles being homogeneously distributed within the feedstock; and
   (d')converting the feedstock into a 3D object.

2. The method of claim 1, wherein the feedstock comprises a filament adapted for 3D printing.

3. The method according to any of claims 1 to 2, wherein the one or more sequence-controlled polymer comprises DNA, or consists of DNA.

4. The method according to any of claims 1 to 3, wherein

   • step (a) further comprises applying at least one of a fountain code, preferably a DNA fountain encoding, and a Reed-Solomon code; and / or
   • step (c) comprises sol-gel synthesis.

5. The method according to any of claims 1 to 4, wherein

   • the plurality of particles comprise silica beads, preferably with diameter of at most 1000 nm; and/or
   • the plurality of particles have a concentration of at most IOO mg/kg within the feedstock; and / or
   • the plurality of particles have a concentration of at most 2mg/g of DNA; and /or
   • the feedstock comprises a thermo-polymer, particularly a polycaprolactone (PCL), polymethylmethacrylate (PMMA), polylactic acid (PLA), acrylonitrile butadien styrene (ABS) or poly(lactic-co-glycolic acid) (PLGA) or a combination thereof.

6. The method of claim 2, wherein converting the feedstock into a 3D object comprises step (e),
   (e) 3D printing an object using the filament; particularly wherein the data comprises a 3D model of the object.

7. A method for recovering information from a 3D object comprising a thermo-polymer and embedded therein a plurality of particles comprising one or more sequence-controlled polymers, said plurality of particles being homogeneously distributed within the thermo-polymer; and
   the method comprising the steps of:

   (f) releasing a plurality of said particles from said thermo-polymer;
   (g) extracting one or more sequence-controlled polymer from said plurality of particles;
   (h) sequencing said one or more sequence-controlled polymer;
   (i) decoding output data from said one or more sequence-controlled polymer, wherein the output data comprises a payload and an error correction code; and
   (j) applying the error correction to the payload.

**8.** The method of claim 7, wherein

- step (f) comprises applying a solvent, preferably tetrahydrofuran, to said thermo-polymer; and/or
- step (g) comprises applying a buffered oxide etch to said plurality of particles.

**9.** The method according to any of claims 7 to 8, wherein the one or more sequence-controlled polymer comprises DNA, or consists of DNA.

**10.** The method according to any of claims 7 to 9, wherein the error correction code comprises at least one of a fountain code, preferably a DNA Fountain code, and a Reed-Solomon code.

**11.** The method according to any of claims 7 to 9, wherein

- the plurality of particles comprise silica beads, preferably with diameter of at most 1000 nm; and/or
- the plurality of particles have a concentration of at most IOO mg/kg within the thermo-polymer; and / or
- the plurality of particles have a concentration of at most 2mg/g of DNA; and / or
- the thermo-polymer comprises polycaprolactone (PCL), polymethylmethacrylate (PMMA), polylactic acid (PLA), acrylonitrile butadien styrene (ABS) or poly(lactic-co-glycolic acid) (PLGA) or a combination thereof.

**12.** A data storage medium comprising a plurality of particles embedded in a thermo-polymer, with said plurality of particles being homogeneously distributed within the thermo-polymer;

- wherein one or more sequence-controlled polymer, preferably comprising DNA, are encapsulated in said plurality of particles; and
- wherein said one or more sequence-controlled polymer encodes predetermined data; and
- wherein said predetermined data comprise a payload and an error correction code.

**13.** The data storage medium of claim 12, having a predetermined shape, wherein the predetermined data comprises a 3D model of the predetermined shape.

**14.** The data storage medium according to any of claims 12 to 13, wherein the error correction code comprises at least one of a fountain code, preferably a DNA Fountain code, and a Reed-Solomon code.

**15.** The data storage medium according to any of claims 12 to 14, wherein

- the plurality of particles comprises silica beads, preferably with a diameter of at most IOOOnm; and / or
- the plurality of particles have a concentration of at most IOOmg/kg within the thermo-polymer; and / or
- the plurality of particles have a concentration of at most 2mg/g of DNA; and / or
- the thermo-polymer comprises polycaprolactone (PCL), polymethylmethacrylate (PMMA), polylactic acid (PLA), acrylonitrile butadien styrene (ABS) or poly(lactic-co-glycolic acid) (PLGA) or a combination thereof.

**Patentansprüche**

**1.** Ein Verfahren zum Speichern von Daten in einem 3D-Objekt unter Verwendung eines datenhaltigen Ausgangsmaterials, wobei das Verfahren die folgenden Schritte umfasst:

(a) Codieren von Eingabedaten in ein oder mehrere sequenzgesteuerte Polymere, wobei das Codieren der Eingabedaten das Anwenden eines Fehlerkorrekturcodes umfasst;
(b) Synthetisieren des einen oder der mehreren sequenzgesteuerten Polymere;
(c) Einkapseln des synthetisierten einen oder der synthetisierten mehreren sequenzgesteuerten Polymere in eine Vielzahl von Partikeln;
(d) Einbetten der Vielzahl von Partikeln in ein Ausgangsmaterial, wobei die Vielzahl von Partikeln homogen innerhalb des Ausgangsmaterials verteilt ist; und
(d') Umwandeln des Ausgangsmaterials in ein 3D-Objekt.

**2.** Das Verfahren nach Anspruch 1, wobei das Ausgangsmaterial ein für den 3D-Druck geeignetes Filament umfasst.

**3.** Das Verfahren nach einem der Ansprüche 1 bis 2, wobei das eine oder die mehreren sequenzgesteuerten Polymere DNA umfasst/umfassen oder aus DNA besteht/bestehen.

**4.** Das Verfahren nach einem der Ansprüche 1 bis 3, wobei

• Schritt (a) weiter das Anwenden mindestens eines von einem Fountain-Code, vorzugsweise einer DNA-Fountain-Kodierung, und einem Reed-Solomon-Code umfasst; und/oder
• Schritt (c) eine Sol-Gel-Synthese umfasst.

**5.** Das Verfahren nach einem der Ansprüche 1 bis 4, wobei

• die Vielzahl von Partikeln Silica-Kügelchen umfasst, vorzugsweise mit einem Durchmesser von höchstens 1000 nm; und/oder
• die Vielzahl von Partikeln eine Konzentration von höchstens 100 mg/kg innerhalb des Ausgangsmaterials aufweist; und/oder
• die Vielzahl von Partikeln eine Konzentration von höchstens 2 mg/g DNA aufweist; und/oder
• das Ausgangsmaterial ein Thermopolymer umfasst, insbesondere Polycaprolacton (PCL), Polymethylmethacrylat (PMMA), Polymilchsäure (PLA), Acrylnitril-Butadien-Styrol (ABS) oder Poly(milchsäure-co-glykolsäure) (PLGA) oder eine Kombination davon.

**6.** Das Verfahren nach Anspruch 2, wobei das Umwandeln des Ausgangsmaterials in ein 3D-Objekt den Schritt (e) umfasst,
(e) 3D-Drucken eines Objekts unter Verwendung des Filaments; insbesondere wobei die Daten ein 3D-Modell des Objekts umfassen.

**7.** Ein Verfahren zur Gewinnung von Informationen aus einem 3D-Objekt, das ein Thermopolymer und darin eingebettete Partikel umfasst, die ein oder mehrere sequenzgesteuerte Polymere enthalten, wobei die Partikel homogen im Thermopolymer verteilt sind; und wobei
das Verfahren die folgenden Schritte umfasst:

(f) Freisetzen einer Vielzahl der Partikel aus dem Thermopolymer;
(g) Extrahieren eines oder mehrerer sequenzgesteuerter Polymere aus der Vielzahl von Partikeln;
(h) Sequenzieren des einen oder der mehreren sequenzgesteuerten Polymere;
(i) Dekodieren von Ausgabedaten aus dem einen oder den mehreren sequenzgesteuerten Polymeren, wobei die Ausgabedaten eine Nutzlast und einen Fehlerkorrekturcode umfassen; und
(j) Anwenden der Fehlerkorrektur auf die Nutzlast.

**8.** Das Verfahren nach Anspruch 7, wobei

• Schritt (f) das Aufbringen eines Lösungsmittels, vorzugsweise Tetrahydrofuran, auf das Thermopolymer umfasst; und/oder
• Schritt (g) das Aufbringen einer gepufferten Oxidätzung auf die Vielzahl von Partikeln umfasst.

**9.** Das Verfahren nach einem der Ansprüche 7 bis 8, wobei das eine oder mehrere sequenzgesteuerte Polymere DNA umfasst oder aus DNA besteht.

**10.** Das Verfahren nach einem der Ansprüche 7 bis 9, wobei der Fehlerkorrekturcode mindestens einen von einem Fountain-Code, vorzugsweise einem DNA-Fountain-Code, und einem Reed-Solomon-Code umfasst.

**11.** Das Verfahren nach einem der Ansprüche 7 bis 9, wobei

• die Vielzahl von Partikeln Silica-Kügelchen umfasst, vorzugsweise mit einem Durchmesser von höchstens 1000 nm; und/oder
• die Vielzahl von Partikeln eine Konzentration von höchstens 100 mg/kg innerhalb des Thermopolymers aufweist; und/oder
• die Vielzahl von Partikeln eine Konzentration von höchstens 2 mg/g DNA aufweist; und/oder
• das Thermopolymer Polycaprolacton (PCL), Polymethylmethacrylat (PMMA), Polymilchsäure (PLA), Acrylnitril-Butadien-Styrol (ABS) oder Poly(milchsäure-co-glykolsäure) (PLGA) oder eine Kombination davon umfasst.

**12.** Ein Datenspeichermedium, umfassend eine Vielzahl von Partikeln, die in ein Thermopolymer eingebettet sind, wobei die Vielzahl von Partikeln homogen innerhalb des Thermopolymers verteilt ist;

• wobei ein oder mehrere sequenzgesteuerte Polymere, vorzugsweise umfassend DNA, in der Vielzahl von Partikeln eingekapselt sind; und
• wobei das eine oder die mehreren sequenzgesteuerten Polymere vorbestimmte Daten kodieren; und
• wobei die vorbestimmten Daten eine Nutzlast und einen Fehlerkorrekturcode umfassen.

**13.** Das Datenspeichermedium nach Anspruch 12, das eine vorbestimmte Form aufweist, wobei die vorbestimmten Daten ein 3D-Modell der vorbestimmten Form umfassen.

**14.** Das Datenspeichermedium nach einem der Ansprüche 12 bis 13, wobei der Fehlerkorrekturcode mindestens einen von einem Fountain-Code, vorzugsweise einem DNA-Fountain-Code, und einem Reed-Solomon-Code umfasst.

**15.** Das Datenspeichermedium nach einem der Ansprüche 12 bis 14, wobei

• die Vielzahl von Partikeln Silica-Kügelchen umfasst, vorzugsweise mit einem Durchmesser von höchstens 1000 nm; und/oder
• die Vielzahl von Partikeln eine Konzentration von höchstens 100 mg/kg innerhalb des Thermopolymers aufweist; und/oder
• die Vielzahl von Partikeln eine Konzentration von höchstens 2 mg/g DNA aufweist; und/oder
• das Thermopolymer Polycaprolacton (PCL), Polymethylmethacrylat (PMMA), Polymilchsäure (PLA), Acrylnitril-Butadien-Styrol (ABS) oder Poly(milchsäure-co-glykolsäure) (PLGA) oder eine Kombination davon umfasst.

**Revendications**

**1.** Un procédé de stockage de données dans un objet 3D à l'aide d'une matière première contenant des données, le procédé comprenant les étapes suivantes :

(a) encodage des données d'entrée dans un ou plusieurs polymères à séquence contrôlée, l'encodage des données d'entrée comprenant l'application d'un code de correction d'erreurs ;
(b) synthétiser le ou les polymères à séquence contrôlée ;
(c) encapsuler le ou les polymères à séquence contrôlée synthétisés dans une pluralité de particules ;
(d) incorporer la pluralité de particules dans une matière première, la pluralité de particules étant répartie de manière homogène au sein de la matière première ; et
(d') convertir la matière première en un objet 3D.

**2.** Le procédé selon la revendication 1, dans lequel la matière première comprend un filament adapté à l'impression 3D.

**3.** Le procédé selon l'une quelconque des revendications 1 à 2, dans lequel le ou les polymères à séquence contrôlée comprennent de l'ADN, ou sont constitués d'ADN.

**4.** Le procédé selon l'une quelconque des revendications 1 à 3, dans lequel

• l'étape (a) comprend en outre l'application d'au moins l'un parmi un code fontaine, de préférence un encodage fontaine d'ADN, et un code de Reed-Solomon ; et/ou
• l'étape (c) comprend une synthèse sol-gel.

**5.** Le procédé selon l'une quelconque des revendications 1 à 4, dans lequel

• la pluralité de particules comprend des billes de silice, de préférence d'un diamètre d'au plus 1000 nm ; et/ou
• la pluralité de particules a une concentration d'au plus 100 mg/kg dans la matière première ; et/ou
• la pluralité de particules a une concentration d'au plus 2 mg/g d'ADN ; et/ou
• la matière première comprend un thermopolymère, en particulier de la polycaprolactone (PCL), du polyméthacrylate de méthyle (PMMA), de l'acide polylactique (PLA), de l'acrylonitrile-butadiène-styrène (ABS) ou de l'acide poly(lactique-co-glycolique) (PLGA) ou une combinaison de ceux-ci.

6. Le procédé selon la revendication 2, dans lequel convertir la matière première en un objet 3D comprend l'étape (e), (e) impression 3D d'un objet à l'aide du filament ; en particulier, dans laquelle les données comprennent un modèle 3D de l'objet.

7. Un procédé de récupération d'informations à partir d'un objet 3D comprenant un thermopolymère et, incorporées dans celui-ci, une pluralité de particules comprenant un ou plusieurs polymères à séquence contrôlée, ladite pluralité de particules étant répartie de manière homogène au sein du thermopolymère ; et
le procédé comprenant les étapes suivantes :

(f) libérer une pluralité desdites particules dudit thermopolymère ;
(g) extraire un ou plusieurs polymères à séquence contrôlée de ladite pluralité de particules ;
(h) séquencer ledit ou lesdits polymères à séquence contrôlée ;
(i) décoder les données de sortie provenant dudit ou desdits polymères à séquence contrôlée, les données de sortie comprenant une charge utile et un code de correction d'erreurs ; et
(j) appliquer la correction d'erreur à la charge utile.

8. Le procédé selon la revendication 7, dans lequel

• l'étape (f) comprend l'application d'un solvant, de préférence du tétrahydrofurane, audit thermopolymère ; et/ou
• l'étape (g) comprend l'application d'une gravure à l'oxyde tamponné sur ladite pluralité de particules.

9. Le procédé selon l'une quelconque des revendications 7 à 8, dans lequel le ou les polymères à séquence contrôlée comprennent de l'ADN, ou sont constitués d'ADN.

10. Le procédé selon l'une quelconque des revendications 7 à 9, dans lequel le code de correction d'erreurs comprend au moins l'un parmi un code fontaine, de préférence un code fontaine d'ADN, et un code de Reed-Solomon.

11. Le procédé selon l'une quelconque des revendications 7 à 9, dans lequel

• la pluralité de particules comprend des billes de silice, de préférence d'un diamètre d'au plus 1000 nm ; et/ou
• la pluralité de particules a une concentration d'au plus 100 mg/kg dans le thermopolymère ; et/ou
• la pluralité de particules a une concentration d'au plus 2 mg/g d'ADN ; et/ou
• le thermopolymère comprend de la polycaprolactone (PCL), du polyméthacrylate de méthyle (PMMA), de l'acide polyactique (PLA), de l'acrylonitrile-butadiène-styrène (ABS) ou de l'acide poly(lactique-co-glycolique) (PLGA) ou une combinaison de ceux-ci.

12. Un support de stockage de données comprenant une pluralité de particules incorporées dans un thermopolymère, ladite pluralité de particules étant répartie de manière homogène au sein du thermopolymère ;

• dans lequel un ou plusieurs polymères à séquence contrôlée, comprenant de préférence de l'ADN, sont encapsulés dans ladite pluralité de particules ; et
• dans lequel ledit ou lesdits polymères à séquence contrôlée codent des données prédéterminées ; et
• dans lequel lesdites données prédéterminées comprennent une charge utile et un code de correction d'erreurs.

13. Le support de stockage de données selon la revendication 12, ayant une forme prédéterminée, dans lequel les données prédéterminées comprennent un modèle 3D de la forme prédéterminée.

14. Le support de stockage de données selon l'une quelconque des revendications 12 à 13, dans lequel le code de correction d'erreurs comprend au moins l'un parmi un code fontaine, de préférence un code fontaine d'ADN, et un code de Reed-Solomon.

15. Le support de stockage de données selon l'une quelconque des revendications 12 à 14, dans lequel

• la pluralité de particules comprend des billes de silice, de préférence d'un diamètre d'au plus 1000 nm ; et/ou
• la pluralité de particules présente une concentration d'au plus 100 mg/kg dans le thermopolymère ; et/ou

• la pluralité de particules présente une concentration d'au plus 2 mg/g d'ADN ; et/ou
• le thermopolymère comprend de la polycaprolactone (PCL), du polyméthacrylate de méthyle (PMMA), de l'acide polylactique (PLA), de l'acrylonitrile-butadiène-styrène (ABS) ou de l'acide poly(lactique-co-glycolique) (PLGA) ou une combinaison de ceux-ci.

Fig. 1

Fig. 3

Fig. 2

Fig. 8

Fig. 4A

```
F0       5' ACACGACGCTCTTCCGATCT
R0       5' AGACGTGTGCTCGGCCGATCT

F1       5' ACACTCTTTCCCTACACGACGCTCTTCCGATCT
R1       5' GTGACTGGAGTTCAGACGTGTGCTCGGCCGATCT

2FU      5' AATGATACGGCGACCACCGAGATCTACACTCTTTCCCTACACGACGC
Index 1  5' CAAGCAGAAGACGGCATACGAGATCACTGTGTGACTGGAGTTCAGACGTGT
Index 2  5' CAAGCAGAAGACGGCATACGAGATATTGGCGTGACTGGAGTTCAGACGTGT
Index 3  5' CAAGCAGAAGACGGCATACGAGATGATCTGGTGACTGGAGTTCAGACGTGT
Index 4  5' CAAGCAGAAGACGGCATACGAGATTCAAGTGTGACTGGAGTTCAGACGTGT
Index 5  5' CAAGCAGAAGACGGCATACGAGATCTGATCGTGACTGGAGTTCAGACGTGT
Index 6  5' CAAGCAGAAGACGGCATACGAGATAAGCTAGTGACTGGAGTTCAGACGTGT
```

Fig. 4B

Fig. 4C

Fig. 5A

Fig. 5B

Fig. 6

Fig. 7   702   701

Fig. 9

Fig. 10

Fig. 15

Fig. 11

Fig. 12

Fig. 13

Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9400910 B2

- US 20190020353 A1

**Non-patent literature cited in the description**

- **GRASS et al.** *Angew. Chem. Int. Ed*, 2015, vol. 54, 2552-2555

- **ERLICH et al.** *Science*, 2017, vol. 355, 950-954
- **PAUNESCU et al.** *Nat. Protocols.*, 2012